# EUROPÄISCHE PATENTSCHRIFT

(11) **EP 0 749 741 B1**
(45) Veröffentlichungstag und Bekanntmachung des Hinweises auf die Patenterteilung: **08.09.1999**
(21) Anmeldenummer: 96810411.7
(22) Anmeldetag: 20.06.1996
(51) Int. Cl.: A61F 13/26

(54) **Tampon-Applikator**
Tampon applicator
Applicateur pour tampons

(30) Priorität: 22.06.1995 CH 183295
(43) Veröffentlichungstag der Anmeldung: 27.12.1996
(73) Patentinhaber: Medivice Limited Company, Georgetown, Cayman Islands, West Indies (KY)
(72) Erfinder: Cimber, Hugo, Dr. Med., 3072 Ostermundigen (CH)
(74) Vertreter: BOVARD AG - Patentanwälte

(56) Entgegenhaltungen:
- DE-A- 3 248 152
- US-A- 4 690 671

## Beschreibung

Die vorliegende Erfindung bezieht sich auf einen Tampon-Applikator gemäss dem Oberbegriff des Patentanspruches 1.

Derartige Tampon-Applikatoren sind bekannt. So zeigt beispielsweise die DE-A-32 48 152 eine entsprechende Einrichtung, bei welcher vor dem Tampon-Kopf in der Einführhülse eine geschlossene Kammer angeordnet ist, was beispielsweise durch Aufstecken oder Aufschrauben eines Zusatzbehälters erfolgen kann, welche mit einem medikamentösen Mittel gefüllt ist. Die durch den Behälter gebildete Kammer ist mindestens gegen die Einführhülse hin mit einer Folie abgeschlossen.

Um zuzulassen, dass das in der Kammer enthaltene medikamentöse Mittel mit dem in der Einführhülse sich befindenden Tampon in Kontakt kommt, was vor dem Einführen oder beim Einführen des Tampons in die Vagina erfolgen soll, muss die dem Tampon-Kopf zugewandte Folie des Behälters aufgebrochen werden. Dies kann einerseits durch relatives Verschieben des Behälters bezüglich der Einführhülse erfolgen, wodurch die Folie durch die Einführhülse aufgebrochen wird, oder andererseits durch das Vorschieben des Tampons während des Einführens durch die Ausstosshülse. Insbesondere im zweiten Fall darf die Folie nur mehr einen schwachen Widerstand gegen das Durchbrechen aufweisen, da sonst die weiche Oberfläche des Tampons nicht fähig ist, die Folie zu durchstossen. Deshalb ist vorgesehen, dass die Folie vorgeprägt ist.

Von Nachteil ist bei dieser Einrichtung, dass durch unbeabsichtigtes Verschieben des Behälters bzw. der Kammer bezüglich der Einführhülse die Trennfolie zum Tampon hin aufgebrochen wird. Ein weiterer Nachteil besteht darin, dass die Folie schwach ausgebildet sein muss, damit sie durch das Vorschieben des Tampons und durch diesen durchbrochen werden kann. Hierbei entsteht die Gefahr, dass diese Folie bereits vor Gebrauch oder vor dem Aufsetzen auf die Einführhülse durch einen geringen mechanischen Einfluss aufgebrochen werden kann, wodurch das darin enthaltene Mittel auslaufen oder zumindest nicht mehr luftdicht verschlossen ist.

Die Aufgabe der vorliegenden Erfindung besteht nun darin, einen Tampon-Applikator zu schaffen, der einen Behälter umfasst, welcher an dem der Einführhülse zugewandten Bereich mit einer Folie verschlossen ist, deren mechanischer Widerstand gegen ungewolltes Aufbrechen genügend gross ist, und die trotzdem durch das Vorstossen des Tampons in der Einführhülse durch die Ausstosshülse während des Einführens des Tampons in die Vagina aufgebrochen werden kann.

Erfindungsgemäss erfolgt die Lösung dieser Aufgabe durch die in der Kennzeichnung des Anspruches 1 angegebenen Merkmale.

In vorteilhafter Weise bestehen die Mittel zum Aufbrechen der Folie aus schwenkbaren Klappen, die innenseitig an der Oberfläche der Einführhülse schwenkbar befestigt sind und die scharfkantig vorstehende Stege oder spitze Zacken aufweisen.

Eine weitere vorteilhafte Ausgestaltung der Erfindung besteht darin, dass die Einführhülse und die schwenkbaren Klappen in einem Stück, vorzugsweise aus Kunststoff, gefertigt sind, und die schwenkbare Verbindung zwischen Klappen und Einführhülse folienartig ausgebildet ist. Dadurch ergibt sich eine einfache und kostengünstige Fertigung der Einführhülse.

Eine weitere vorteilhafte Ausgestaltung der Erfindung besteht darin, dass die Aufnahmemittel für den Behälter aus einem an der Einführhülse angeformten zylinderförmigen Bereich bestehen, in welche der Behälter einsetzbar ist. Hierzu ist ein Steg vorgesehen, welcher für den eingesetzten Behälter als Anschlag dient, wobei Schnappmittel vorgesehen sind, welche den eingesetzten Behälter gegen den Anschlag festhalten. Dadurch wird erreicht, dass der Behälter nicht zu weit in die Einführhülse eingesetzt werden kann, wodurch ein vorzeitiges Aufbrechen der Folie vermieden wird.

Um ein problemloses Ausstossen des Tampons aus der Einführhülse und durch den aufgesetzten Behälter erreichen zu können, sind in vorteilhafter Weise der Innendurchmesser der Einführhülse und der Innendurchmesser des Behälters im wesentlichen gleich.

Eine Ausführungsform eines erfindungsgemässen Tampon-Applikators wird nachfolgend beispielhaft anhand der beiliegenden Zeichnung näher erläutert.

Es zeigt
Fig. 1 eine Längsschnittdarstellung durch einen erfindungsgemässen Tampon-Applikator mit eingesetztem Tampon;
Fig. 2 eine Schnittdarstellung durch den erfindungsgemässen Tampon-Applikator gemäss Fig. 1, bei welchem der Tampon vorgeschoben und die Folie des Behälters aufgebrochen ist;
Fig. 3 eine Draufsicht auf die Einführungshülse gemäss Fig. 1 und Fig. 2 mit den schwenkbaren Klappen; und
Fig. 4 eine Schnittdarstellung durch eine Klappe entlang Linie IV-IV gemäss Fig. 3.

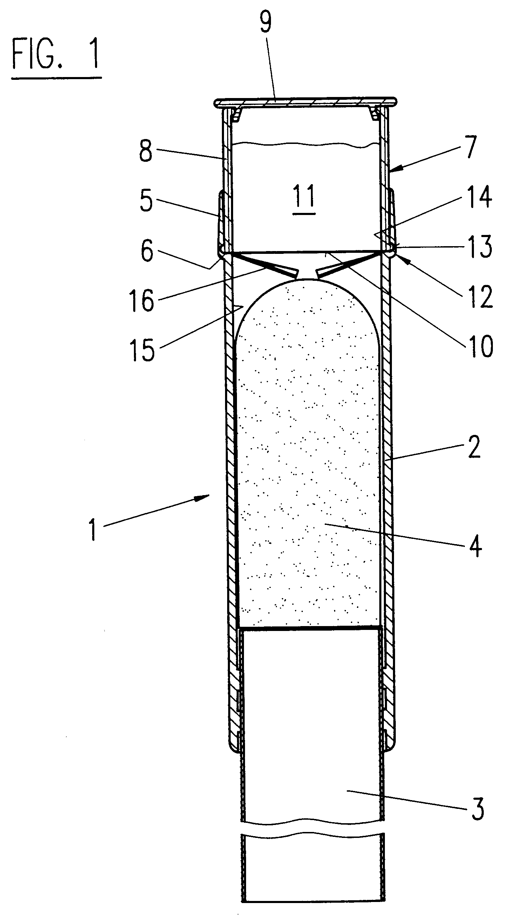

Wie aus Fig. 1 ersichtlich ist, besteht der erfindungsgemässe Tampon-Applikator 1 aus einer hohlzylinderförmigen Einführhülse 2, in welcher eine Ausstosshülse 3 verschiebbar eingesetzt ist. In die Einführhülse ist ein Tampon 4 eingelegt.

An der der Ausstosshülse 3 gegenüberliegenden Seite der Einführhülse 2 ist ein zylinderförmiger Bereich 5 angeformt. Da dieser zylinderförmige Bereich 5 einen geringfügig grösseren Durchmesser aufweist, als die Einführhülse 2, entsteht ein Steg 6.

In den zylinderförmigen Bereich 5 ist ein Behälter 7 einsetzbar, der eine Wandung 8 und einen abnehmbaren Deckel 9 aufweist, und der gegen die Einführhülse 2 hin mit einer Folie 10 verschlossen ist. In diesem Behälter 7 ist ein therapeutisches oder prophylaktisches Medikament 11 enthalten.

Der Behälter 7 wird soweit in den zylinderförmigen Bereich 5 eingeschoben, bis er am Steg 6 ansteht. In dieser Position wird der Behälter 7 durch Schnappmittel 12 festgehalten, die aus einer innenseitig in den zylinderförmigen Bereich 5 eingelassenen Nut 13 bestehen, in welche Nocken 14, die am Behälter 7 angebracht sind, einschnappen können. In diesem Zustand ist der Behälter 7 mit der Einführhülse 2 relativ fest verbunden.

An der innenseitigen Oberfläche 15 der Einführhülse 2 sind schwenkbare Klappen 16 angeordnet, welche in der in Fig. 1 dargestellten Ausgangslage im wesentlichen nach innen von der innenseitigen Oberfläche 15 abstehend gerichtet sind. Die Einführhülse 2 und die Klappen 16 können aus dem gleichen Material, vorzugsweise Kunststoff wie Polyäthylen oder dgl., in einem Arbeitsgang gefertigt werden, wobei die schwenkbare Verbindung zwischen Klappe 16 und Einführhülse 2 folienartig ausgebildet ist.

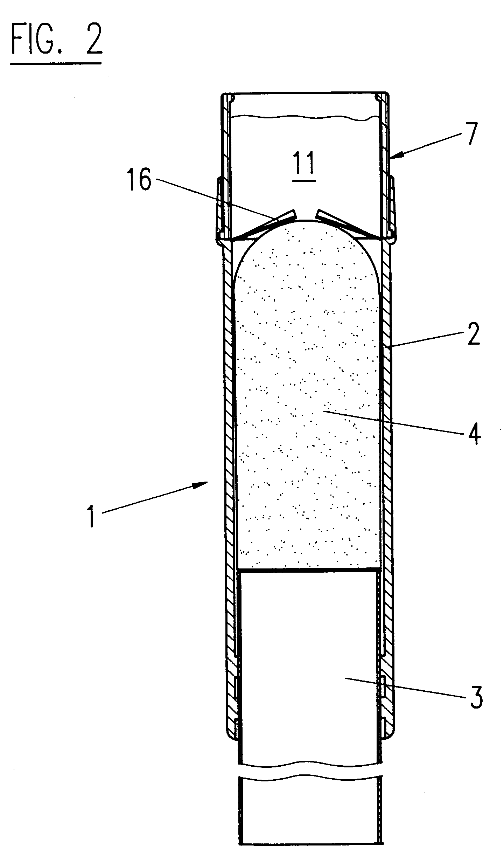

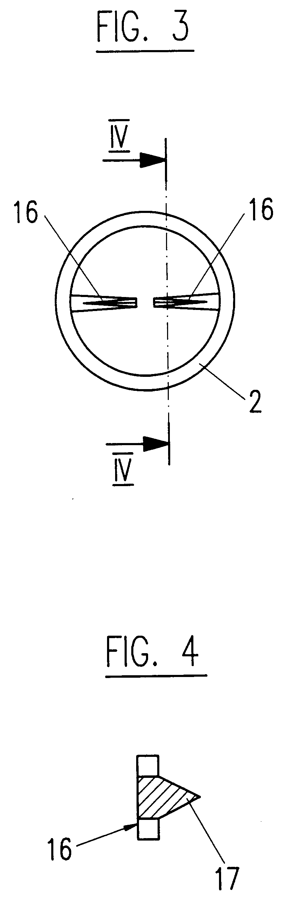

Wie aus Fig. 2 ersichtlich ist, wird zum Einführen des Tampon-Applikators in die Vagina eines weiblichen Körpers der Deckel 9 weggenommen. Der Tampon 4 wird durch die Ausstosshülse 3 gegen den Behälter hin 7 verschoben, wodurch die Klappen 16 gegen die Folie 10 geschwenkt werden. Der scharfkantige vorstehende Steg 17 (Fig. 4) bricht die Folie 10 auf, der Tampon 4 kann weiter vorgeschoben werden, und wird zusammen mit dem Medikament 11 aus dem Tampon-Applikator 1 ausgestossen. Dieses Ausstossen des Tampons 4 mit dem Medikament 11 wird dadurch erleichtert, dass der Innendurchmesser der Einführhülse 2 und der Innendurchmesser des Behälters 7 gleich gross sind.

Selbstverständlich sind alle Kanten der Einführhülse 2 und des Behälters 7 abgerundet, damit Verletzungen beim Einführen in die Vagina vermieden werden.

Aus Fig. 3 ist die Anordnung der schwenkbaren Klappen 16 in der Einführhülse 2 ersichtlich. Im vorliegenden Ausführungsbeispiel sind zwei Klappen 16 vorgesehen, denkbar ist aber auch eine grössere Anzahl.

In der Schnittdarstellung gemäss Fig. 4 ist eine Klappe 16 ersichtlich, die mit einem scharfkantigen Steg 17 ausgestattet ist, welcher gegen die Folie 10 des Behälters 7 gerichtet ist, die aufgebrochen werden soll.

Mit diesem erfindungsgemässen Tampon-Applikator wird eine einfache und sichere Handhabung ermöglicht, wobei die Stärke der Folie 10 so ausgebildet sein kann, dass sie nicht durch ungewollte mechanische Einflüsse aufgebrochen wird, und dass sie trotzdem durch das Vorschieben des Tampons zum Einführen durchbrochen werden kann.

## Patentansprüche

1. Tampon-Applikator (1), welcher eine Einführhülse (2) zur Aufnahme eines Tampons (4) und eine darin verschiebbare Ausstosshülse (3) zum Ausstossen des Tampons umfasst, und die Einführhülse (2) am einführseitigen Endbereich mit Aufnahmemitteln (11) zur Aufnahme eines mit einer Wandung versehenen Behälters ausgestattet ist, in welchem ein medikamentöses Mittel enthalten ist und welcher jeweils stirnseitig verschlossen ist, wobei der der Einführhülse (2) zugewandte Bereich mit einer Folie (10) verschlossen ist, dadurch gekennzeichnet, dass in der Einführhülse (2) zwischen dem eingesetzten Tampon (4) und dem mit der Folie (10) verschlossenen, auf die Einführhülse (2) aufgesetzten Behälter (7) Mittel (16) vorgesehen sind, die durch das Vorstossen des Tampons (4) mit der Ausstosshülse (3) gegen die Folie (10) hin bewegbar sind und diese aufbrechen.

2. Tampon-Applikator nach Anspruch 1, dadurch gekennzeichnet, dass die Mittel zum Aufbrechen der Folie (10) des Behälters (7) aus mindestens einer schwenkbaren Klappe (16) bestehen, welche an der innenseitigen Oberfläche (15) der Einführhülse (2) schwenkbar befestigt ist und in der Ausgangslage im wesentlichen nach innen von der innenseitigen Oberfläche (15) abstehend gerichtet ist.

3. Tampon-Applikator nach Anspruch 2, dadurch gekennzeichnet, dass die Einführhülse (2) und die mindestens eine schwenkbare Klappe (16) in einem Stück gefertigt sind, wobei die schwenkbare Verbindung zwischen Klappe (16) und Einführhülse (2) folienartig ausgebildet ist.

4. Tampon-Applikator nach Anspruch 2 oder 3, dadurch gekennzeichnet, dass zwei einander gegenüberliegende Klappen (16) vorgesehen sind.

5. Tampon-Applikator nach einem der Ansprüche 2 bis 4, dadurch gekennzeichnet, dass an jeder Klappe (16) auf der der Folie (10) des auf die Einführhülse (2) aufgesetzten Behälters (7) zugewandten Seite mindestens ein scharfkantiger vorstehender Steg (17) oder vorstehende spitze Zacken angeordnet sind.

6. Tampon-Applikator nach einem der Ansprüche 1 bis 5, dadurch gekennzeichnet, dass die Aufnahmemittel für den Behälter (7) in der Einführhülse (2) aus einem an der Einführhülse (2) angeformten zylinderförmigen Bereich (5) bestehen, in welchen der Behälter (7) einsetzbar ist.

7. Tampon-Applikator nach Anspruch 6, dadurch gekennzeichnet, dass der zylinderförmige Bereich (5) gegen die Einführhülse (2) hin mit einem Steg (6) ausgestattet ist, welcher für den eingesetzten Behälter (7) als Anschlag dient, und dass Schnappmittel (12) vorgesehen sind, welche den eingesetzten Behälter (7) festhalten.

8. Tampon-Applikator nach Anspruch 6 oder 7, dadurch gekennzeichnet, dass der Innendurchmesser der Einführhülse (2) und der Innendurchmesser des Behälters (7) im wesentlichen übereinstimmen.

## Claims

1. Tampon applicator (1), which comprises an insertion tube (2) for accepting a tampon (4) and a discharge tube (3), displaceable in said insertion tube, for discharging the tampon, and the insertion tube (2) is provided with receiving means (11) on the end region on the insertion side for receiving a container provided with a walling, in which container a medical substance is contained and which is sealed off each time on the face, the region turned toward the insertion tube (2) being sealed with a foil (10), characterised in that provided in the insertion tube (2) between the inserted tampon (4) and the container (7), sealed with the foil (10), and placed upon the insertion tube (2), are means (16), which are movable by means of the pressing forward of the tampon (4) with the discharge tube (3) against the foil (10), and which break this foil.

2. Tampon applicator according to claim 1, characterised in that the means to break through the foil (10) of the container (7) consist of at least one pivotable flap (16) which is pivotably mounted on the inside surface (15) of the insertion tube (2) and which in the starting position is directed essentially inward, protruding from the inside surface (15).

3. Tampon applicator according to claim 2, characterised in that the insertion tube (2) and the at least one pivotable flap (16) are made in one piece, the pivotable connection between the flap (16) and the insertion tube (2) being of foil-type construction.

4. Tampon applicator according to claim 2 or 3, characterised in that two flaps (16) are provided, opposite each other.

5. Tampon applicator according to one of the claims 2 to 4, characterised in that at least one sharp-edged protruding ridge (17) or protruding pointed teeth are disposed on each flap (16) on the side turned toward the foil (10) of the container (7) placed upon the insertion tube (2).

6. Tampon applicator according to one of the claims 1 to 5, characterised in that the receiving means for the container (7) in the insertion tube (2) comprise a cylindrical region (5) formed on the insertion tube (2) into which the container (7) is insertable.

7. Tampon applicator according to claim 6, characterised in that the cylindrical region (5) is provided with a ridge (6) toward the insertion tube (2), which ridge serves as a limit stop for the inserted container (7), and in that snap means (12) are provided which hold firmly the inserted container (7).

8. Tampon applicator according to claim 6 or 7, characterised in that the inner diameter of the insertion tube (2) and the inner diameter of the container (7) essentially correspond.

## Revendications

1. Applicateur de tampons (1), comprenant un manchon d'introduction (2) pour recevoir un tampon (4) et une douille de poussée (3) mobile dans le manchon d'introduction, apte à pousser le tampon, le manchon d'introduction (2) étant muni, à son extrémité d'introduction, d'un réceptacle (11) pour recevoir un récipient muni d'une paroi contenant un produit médicamenteux, chaque face du récipient étant fermée, la portion tournée vers le manchon d'introduction (2) étant fermée par une feuille (10), caractérisé en ce que des moyens (16) sont prévus dans le manchon d'introduction (2) entre le tampon (4) qui y est introduit et le récipient (7) disposé sur le manchon d'introduction (2) fermé par la feuille (10), étant aptes à se déplacer contre la feuille (10) et à la déchirer sous l'action d'une poussée du tampon (4) par la douille de poussée (3).

2. Applicateur de tampon selon la revendication 1, caractérisé en ce que les moyens permettant de déchirer la feuille (10) du récipient (7) comprennent au moins un clapet (16) pivotant fixé de manière pivotante sur la paroi interne (15) du manchon d'introduction (2) et dirigé en position de repos essentiellement vers l'intérieur de la paroi interne (15).

3. Applicateur de tampon selon la revendication 2, caractérisé en ce que le manchon d'introduction (2) et ledit ou lesdits clapets pivotants (16) sont constitués en une seule pièce, la liaison pivotante entre le clapet (16) et le manchon d'introduction (2) étant formée d'une feuille.

4. Applicateur de tampon selon l'une des revendications 2 ou 3, caractérisé en ce que deux clapets (16) opposés sont prévus.

5. Applicateur de tampon selon l'une des revendications 2 à 4, caractérisé en ce qu'au moins un profil acéré (17) ou des dents pointures en saillie sont disposés sur la face de chaque clapet (16) tournée vers la feuille (10) du récipient (7) contenu dans le manchon d'introduction (2).

6. Applicateur de tampon selon l'une des revendications 1 à 5, caractérisé en ce que les réceptacles pour le récipient (7) dans le manchon d'introduction (2) consistent en une région (5) de forme cylindrique formée dans le manchon d'introduction (2), dans laquelle le récipient (7) peut être logé.

7. Applicateur de tampon selon la revendication 6, caractérisé en ce que la région (5) de forme cylindrique est munie d'un épaulement (6) contre le manchon d'introduction (2), servant de butée au récipient (7) qui y est logé, des moyens d'enclenchement (12) étant prévus afin de fixer le récipient (7) qui y est logé.

8. Applicateur de tampon selon l'une des revendications 6 ou 7, caractérisé en ce que le diamètre intérieur du manchon d'introduction (2) et le diamètre intérieur du récipient (7) essentiellement concordent.
